# EUROPÄISCHE PATENTSCHRIFT

(11) **EP 2 525 768 B1**
(45) Veröffentlichungstag und Bekanntmachung des Hinweises auf die Patenterteilung: **07.05.2014**
(21) Anmeldenummer: 11700617.1
(22) Anmeldetag: 19.01.2011
(51) Int. Cl.: A61K 6/00, A61L 24/04

(54) **HAFTCREME FÜR ZAHNPROTHESEN**
ADHESIVE CREAM FOR DENTAL PROSTHESES
CRÈME ADHÉSIVE POUR DES PROTHÈSES DENTAIRES

(30) Priorität: 06.05.2010 CH 701102010; 19.01.2010 CH 74102010
(43) Veröffentlichungstag der Anmeldung: 28.11.2012
(73) Patentinhaber: Bogaert, Jean Pierre, 98000 Monte Carlo (MC)
(72) Erfinder: Bogaert, Jean Pierre, 98000 Monte Carlo (MC)
(74) Vertreter: Hasler, Erich
(86) Internationale Anmeldenummer: PCT/EP2011/000196
(87) Internationale Veröffentlichungsnummer: WO 2011/088989

(56) Entgegenhaltungen:
- EP-A1- 0 048 556
- US-A- 5 561 177
- US-A1- 2004 141 933

## Beschreibung

### Gebiet der Erfindung

Die Erfindung betrifft Haftcremezusammensetzungen gemäss der Oberbegriffe der Ansprüche 1 und 3.

### Stand der Technik

Viele kommerzielle Haftcremezusammensetzungen für Zahnprothesen basieren auf einem Gemisch von raffinierten Paraffinen, wasserlöslichen Cellulosederivat-Polymeren und Alkylvinylether/Maleinsäureanhydrid-Copolymeren Für gewöhnlich werden Mineralöle und mineralische Fette, insbesondere Vaseline verwendet. Mineralöle und Fette machen üblicherweise ca. 40 Gewichtsprozent (Gew.-%) oder mehr der Haftcremezusammensetzung aus.

Da sich eine Haftcreme während ihrer Anwendung im Mund langsam auflöst, besteht zum einen die Möglichkeit, dass Bestandteile der Creme über die Mund- und Rachenschleimhaut in den Körper gelangen, und zum anderen gelangen Bestandteile mit dem Speichel und der Nahrung in den Verdauungstrakt und verteilen sich danach möglicherweise im ganzen Körper. Die Basisbestandteile Vaseline und Mineralöl sind nach neuesten Forschungsergebnissen nicht völlig unbedenklich. Vaseline zum Beispiel scheint bei Neugeborenen Hefepilzinfektionserkrankungen zu fördern. Negative Auswirkungen bei Erwachsenen sind somit nicht auszuschliessen. Besonders bei der regelmässigen Anwendung von Mineralölen auf Schleimhäuten scheint Vorsicht geboten zu sein. Ein Ersatz für Mineralöle sowie mineralische Fette wäre daher wünschenswert.

Zur Haftungsverbesserung werden üblicherweise zinlchaltige Substanzen zugegeben. Solche kommerziellen Haftcremeprodukte weisen gemäss hauseigenen Analysen einen Zinkgehalt von 1.7 bis 3.4 Gew.-% auf. In Dokument US 4,758,630 wird ein Zinkgehalt von 1 bis 2.4 Gew.-% der Gesamtmenge der Haftcremezusammensetzung empfohlen. In jüngerer Zeit wurde ebenfalls bekannt, dass eine grössere Aufnahme von Zinkverbindungen durch den Organismus, das bislang als unbedenklich galt, teilweise irreversible Vergiftungssymptome verursacht. Bei regelmässiger Anwendung könnten die Zinkverbindungen in der Haftcreme also ähnliche Auswirkungen haben. Um toxische Symptome bei Zahnprothesenträgern zu verhindern, besteht ein Bedarf an einer Haftcremeformulierung, welche auch bei reduziertem oder ausgeschlossenem Zusatz von Zinkverbindungen einwandfreie Trageeigenschaften und Lagerfähigkeit aufweist.

Darüber hinaus enthalten kommerzielle Ausgangsprodukte, die in der Haftcremeherstellung verwendet werden, ohnehin Spuren von Zinkverunreinigungen wie z.B. Gantrez ® MS 955, ein Methylvinylether/Maleinsäureanhydrid-Copolymer, welches häufig in der Herstellung von Haftcremes benutzt wird. Analysen haben ergeben, dass dieses Ausgangsprodukt 4.8 Milligramm Zink pro Kilogramm enthält. Daraus resultiert, dass in einer damit hergestellten Haftcreme ein Gehalt von ca. 2 ppm Zink, maximal jedoch 4.8 ppm Zink zu erwarten ist. Der Anteil an Zinkverbindungen, der in solchen Ausgangsprodukten enthalten sein kann, ist jedoch weitaus geringer als jener, der - wie oben erwähnt - in kommerziellen Haftcremes gemessen wird und aus gezielter Zugabe stammt

Könnten nun bestehende Formulierungen mit Methylvinylether/Maleinsäureanhydrid-Copolymer derart abgeändert werden, dass auf Mineralöle und/oder Vaseline und zusätzlich zugegebene Zinkverbindungen teilweise oder ganz verzichtet werden kann, würde ein Produkt entstehen, das als gesundheitlich bedenkenlos betrachtet werden kann. Insbesondere die Verwendung von pflanzlichen Ölen und Fetten würde dem heutigen Gesundheitsbewusstsein der Konsumenten entgegenkommen.

Die Inhaltsstoffe und deren Mengenverhältnisse bestimmen die Eigenschaften einer Haftcreme. Zu den Eigenschaften zählen zum Beispiel die Haftungsstärke, die Haftungsdauer, der Tragekomfort, der Geschmack, die Konsistenz, die Stabilität usw. Zur Einstellung der Haftungsparameter wie Haftungsdauer und Haftungsstärke wird bei kommerziellen Produkten auf Mineralölbasis ein gewisser Anteil Zn²⁺-Verbindungen zugegeben. Durch Zugabe von Zinkverbindungen werden gemäss Dokument US 4,758,630 die Haftkraft und die Haftungsdauer positiv beeinflusst und der Zusatz von Zinkverbindungen ist somit für die Gebrauchsfähigkeit der Haftcreme von herausragender Bedeutung.

Wie oben erwähnt werden in vielen kommerziellen Haftcremes Paraffine wie raffinierte mineralische Öle und Fette (Vaseline) verwendet. Dies sind Gemische aus gesättigten Kohlenwasserstoffen mit der allgemeinen Summenformel CₙH₂ₙ₊₂, wobei es sich je nach Destillationssgrad um flüssige (Öle) bzw. schmierige bis feste Produkte (Fette) handelt. Auch die in der Kosmetik und Medizin Verwendung findenden Mineralölgemische und Fette, bestehen ebenfalls praktisch ausschliesslich aus gesättigten Kohlenwasserstoffen. Die Reinheit der Gemische richtet sich nach dem Raffinationsgrad. Für die kosmetische und medizinische Anwendung ist der Raffinationsgrad hoch. Dadurch soll sichergestellt werden, dass karzinogen wirkende polyzyklische aromatische Kohlenwasserstoffe möglichst vollständig aus dem Gemisch entfernt sind.

Mineralöle und Fette werden häufig als Grundlagen von Hautcremes verwendet, wobei deren Wirkung hier unterschiedlich eingestuft wird. Anerkannte Expertengruppen sind der Meinung, dass Paraffine die natürlichen Regulationsmechanismen des menschlichen Körpers behindern können. Insbesondere können sie sich in Leber, Niere und Lymphknoten anreichern. Es besteht jedoch Unklarheit darüber, ob die Paraffine die Haut penetrieren können. Im Allgemeinen wird davon ausgegangen, dass bei topischer Applikation Paraffine nicht in die Haut penetrieren und daher auch keine Gefährdung von diesen Stoffen ausgeht. Kosmetische Hautcremes mit Ölen und Fetten sind seit vielen Jahren am Markt. Bei der Verwendung in Haftcremes besteht jedoch eine erhöhte Wahrscheinlichkeit, dass paraffinische Bestandteile direkt vom Körper über die Verdauung aufgenommen werden.

In Dokument US 5,561,177 wird eine Haftcremeformulierung offenbart, welche ausgehend von einer Basis pflanzlicher Öle hergestellt wird. Danach sind solche Öle erfolgreich einsetzbar, welche vor allem Triglyceride gesättigter Fettsäuren enthalten. Zudem besitzen diese Triglyceride Kohlenstoffketten mittlerer Länge, d.h. Ketten mit 8 bzw. 10 C-Atomen im Fettsäurerest, die nicht dem typischen Fetsäurespektrum pflanzlicher Öle und Fette (C₁₂-C₂₀) entsprechen. Vom Gebrauch von Ölen, die ungesättigte Fettsäuren enthalten, wird abgeraten. Gemäss US 5,561,177 haben Öle, die ungesättigte Fettsäureester enthalten, den Nachteil, dass sie bei erhöhten Temperaturen oder nach einer gewissen Lagerung instabil werden. Zudem sind Haftcremes, die solche Öle enthalten, aufgrund der vorhandenen ungesättigten Doppelbindungen weniger fest, d.h. zu flüssig für die Anwendung als Haftcreme.

### Aufgabe der Erfindung

Eine Aufgabe der vorliegenden Erfindung liegt darin, eine Haftcremezusammensetzung zu formulieren, die eine gute Haftkraft und Langzeitstabilität aufweist. Im Weiteren soll die Haftcremezusammensetzung auf vorwiegend natürlichen und physiologisch unbedenklichen Ausgangsprodukten basieren. Insbesondere soll eine Formulierung gefunden werden, welche auch mineralölfrei und frei von zinkhaltigen Verbindungen ist oder gegebenenfalls bei geringem Mineralöl- und/oder Zinkgehalt gute Haftcremeeigenschaften aufweist. Eine weitere Aufgabe ist es, eine in physiologischer Hinsicht verbesserte Haftcreme zu erzeugen, die hinsichtlich ihrer Stabilitäts- und Haftungseigenschaften, insbesondere bezüglich der Haftungsdauer und der Haftungsstärke, im Vergleich mit den kommerziellen Cremes zumindest gleichwertig ist. Im Weiteren wird eine Haftcreme angestrebt, welche ein angenehmes Gefühl im Mund erzeugt.

### Beschreibung

Erfindungsgemäss wird die Aufgabe in einer ersten Ausgestaltung mit einer Haftcremezusammensetzung gemäss Anspruch 1 dadurch gelöst, dass die Zusammensetzung einer Haftcreme, die a) wenigstens ein Öl, oder Fett vorzugsweise pflanzlicher Art, b) wenigstens ein wasserlösliches Polymer ausgewählt aus der Gruppe der Cellulosederivate, und c) wenigtens ein Alkylvinylether/Maleinsäureanhydrid-Copolymer beinhaltet, dadurch gekennzeichnet ist, dass d) Trihydroxystearin darin enthalten ist.

Überraschenderweise wurde festgestellt, dass durch Zugabe von Trihydroxystearin die Hafteigenschaften der Haftcreme verbessert werden.

Weitere Substanzen ausgewählt aus der Gruppe bestehend aus Siliziumdioxid, Phosphoglyceriden und Polyethylenglycolen können vorteilhaft zugegeben werden um die Eigenschaften weiter zu verbessern.

Die Aufgabe wird in einer zweiten Ausgestaltung dadurch gelöst, dass die Zusammensetzung sich weiter dadurch auszeichnet, dass der Fettsäuregehalt (wobei mit Fettsäuregehalt im Nachfolgenden die als Ester gebundene Fettsäure zu verstehen ist) des enthaltenen pflanzlichen Öls und/oder Fetts zumindest zu 20 Gew.-% aus ungesättigten Fettsäuren besteht.

Die erfindungsgemässe Zusammensetzung hat den Vorteil, dass sie aus physiologisch völlig unbedenklichen Komponenten zusammengesetzt ist und daher auch bei längerer Anwendung unbedenklich ist. Überraschenderweise konnte eine Zusammensetzung gefunden werden, die trotz eines hohen Anteils an ungesättigten Fettsäuren eine sehr gute und lang andauernde Haftung gewährleistet. Auch konnte eine gute Lagerstabilität erreicht werden.

Überraschend wurde festgestellt, dass die Fliessfähigkeit und die Konsistenz der Creme durch Zugabe von Siliziumdioxid positiv beeinflusst werden können. Durch Siliziumdioxid kann eine Verflüssigungsneigung wirksam verhindert werden.

In dieser Erfindungsausgestaltung, welche dadurch charakterisiert ist, dass das pflanzliche Öl bzw. Fett einen gewissen minimalen ungesättigten Fettsäureanteil beinhaltet, werden besonders vorteilhafte Hafteigenschaften erzielt, wenn die Zusammensetzung der Haftcreme weitere Substanzen, ausgewählt aus der Gruppe bestehend aus Phosphoglyceriden und Polyethylenglycolen, beinhaltet.

Die folgende Beschreibung bezieht sich auf alle oben aufgezeigte Ausgestaltungen.

Überraschenderweise wurde festgestellt, dass auf den zur Einstellung der Haftungsparameter üblicherweise zugegebenen hohen Anteil an Zinkverbindungen verzichtet werden kann, wenn gleichzeitig ein oder mehrere pflanzliche Öle und/oder Fette verwendet werden. Pflanzliche Öle und Fette werden aus der Saat bzw. Frucht von Ölpflanzen gewonnen. Chemisch sind Pflanzenöle und Fette Ester des Glycerins mit Fettsäuren, häufig mit drei Fettsäuren, sogenannte Triglyceride. Viele pflanzliche Öle werden vom Menschen regelmässig mit der Nahrung aufgenommen und sind in den üblicherweise in der Nahrung vorkommenden Mengen physiologisch völlig unbedenklich. Beispiele für die in der erfindungsgemässen Haftcremezusammensetzung verwendbaren Öle und Fette sind Olivenöl, Rapsöl, Erdnussöl, Maisöl, Weizenkeimöl, Walnussöl, Traubenkernöl, Sonnenblumenöl, Weizenkeimöl, Sesamöl, Palmöl, Palmkernöl, Mohnöl, Leinöl, Kürbiskemöl, Distelöl, Nachtkerzenöl, Hanföl und Kokosnussfett. Hierbei wird Olivenöl bevorzugt verwendet, da dieses zu einer besonders physiologisch vertäglichen und geschmacklich akzeptierten Haftcreme führt unter gleichzeitigem Verzicht des Zusatzes von Zinkverbindungen. Bekanntermassen ist raffiniertes Olivenöl geschmacklich relativ neutral, leicht erhältlich und allgemein als gesund und bekömmlich bekannt. Besonders vorteilhaft wirkt sich beim Olivenöl auch aus, dass dieses antiseptisch und antibakteriell wirkt. In Versuchen hat sich gezeigt, dass Olivenöl überraschenderweise das Wachstum von Keimen (Bakterien, Pilze) zwischen Gaumen und Prothese deutlich verlangsamt. Insbesondere konnte auch festgestellt werden, dass der Pilz Candida albicans mit Hilfe der mit Olivenöl angereicherten Haftcreme bekämpft werden kann.

Besonders überraschende Ergebnisse werden bei der Kombination von pflanzlichen Ölen in Haftcremen mit Siliziumdioxidzusätzen erreicht. Pflanzliche Öle und Fette sind aufgrund ihrer hohen Doppelbindungsanteile flüssig oder zumindest stark fliessfähig. Um ein Wegfliessen der Haftcreme unter der Prothese zu vermindern und gleichzeitig die Haftungseigenschaften zu beeinflussen können verschiedene Stabilisatoren in die Haftcrememischung eingebracht werden. Siliziumdioxid erwies sich als besonders geeignet. Insbesondere wird nicht nur eine lange Haftungsdauer bei konstant guter Haftungsstärke erreicht, sondern es wird auch die Stabilität unter widrigen Umweltbedingungen, wie zum Beispiel bei Transport der Creme im Flugzeug bei Unterdruck oder bei Lagerung in erhöhten Berglagen, gewährt. Siliziumdioxid (SiO₂) ist in verschiedenen Formen und Qualitäten erhältlich. Bevorzugt wird Siliziumdioxid in Form von hochdispersem Siliziumdioxid (d.h. in Form eines mittels Flammverfahren hergestellten amorphen Kieselpulvers) bekannt auch als pyrogene Kieselsäure (Aerosil®) verwendet

Die erfindungsgemässe Haftcreme zeichnet sich somit durch eine gesundheitlich unbedenkliche Zusammensetzung aus, die auf pflanzlichen Ölen und/oder Fetten insbesondere Olivenöl basiert. Olivenöl kann hierbei gleichermassen in teilraffiniertem wie auch unraffiniertem Zustand verwendet werden. Vorteilhafterweise wird kaltgepresstes und ohne übermäßige Temperatureinwirkung schonend hergestelltes Olivenöl aus erster Pressung verwendet (d.h. natives Olivenöl Extra).

Neben dem Siliziumdioxid führt auch Trihydroxystearin und Polyethylenglycol zu stabilen Haftcremezusammensetzungen. Diese Substanzen liefern sowohl für Crems auf Basis pflanzlicher Öle sowie für Crems auf Basis mineralischer Öle gute Ergebnisse.

Die Haftcreme enthält bezogen auf die Gesamtmenge der Zusammensetzung vorteilhafterweise
a) 25-60 Gew.% wenigstens eines Öls und/oder Fetts bevorzugt pflanzlichen Öls und/oder Fetts,
b) 10-40 Gew.-% wenigstens eines wasserlöslichen Polymers ausgewählt aus der Gruppe der Cellulosederivate,
c) 25-45 Gew.-% wenigstens eines Alkylvinylether/Maleinsäureanhydrid-Copolymers,
d) bis zu 2.5 Gew.-% Trihydroxystearin,
e) 0-15 Gew.-% Polyethyleneglycole oder ggf. bis zu 15 Gew.-% Polyethylenglycole,
f) 0-3 Gew.-% Phosphoglyceride oder ggf. bis zu 3 Gew.-% Phosphoglyceride,
g) 0-5 Gew.-% Siliziumdioxid oder ggf. bis zu 5 Gew.-% Siliziumdioxid, und
h) 0-10 Gew.-% weitere Zusatzstoffe.

Die meisten weiteren Zusatzstoffe werden zweckmässigerweise ausgewählt aus der Gruppe der Antioxidantien, Geschmackstoffe, Farbstoffe, Stabilisatoren, Verdicker, Emulgatoren, und aus Gemischen davon. Ein Stoff kann hierbei mehreren Wirkungsgruppen angehören, bzw. mehrere Wirkungen zeigen.

Die Stabilisatoren wirken emulgierend, verdickend und verhindern ein Trennen der Bestandteile. Diese werden erfindungsgemäss aus der Gruppe beinhaltend Siliziumdioxid, Trihydroxystearin, Phosphoglyceride und Polyethylenglycole ausgewählt. Diese Substanzen zeigen komplexe Wirkmechanismen und können sich - gemäss unseren Erfahrungen - gegenseitig synergetisch beeinflussen. Die Stabilität einer Haftcreme ist während der Lagerung und Aufbewahrung und für die Dauer der Anwendung von Bedeutung. Während der Lagerung und Aufbewahrung sind Feuchtigkeit, Temperatur und Druck meist relativ konstant. Für die Dauer der Anwendung hingegen wirken zusätzlich Mundspeichel, Nahrungsmittel und sich verändernde Druck- und Temperaturverhältnisse auf die Haftcreme. Aufgrund dieser sich ändernden Beanspruchung kann es vorteilhaft sein, verschiedene Stabilisatorsubstanzen zuzugeben, die in Summe bei den verschiedenen Umwelteinflüssen die Haftcremeeigenschaften verbessern und insbesondere stabilisieren. Als Stabilisator finden Verdicker Anwendung. Verdicker, auch als verdickende Stabilisatoren bezeichnet, werden bevorzugt in einer Menge von 0.001 bis 3 Gew.-% bezogen auf die Gesamtmenge der Zusammensetzung zugegeben. Zweckmässig ist die Verwendung von Siliziumdioxid in einer Menge von 0.001-5 Gew.-%, bevorzugt von 0.1-4 Gew.-% und weiter bevorzugt von 0.5-3 Gew.-% bezogen auf die Gesamtmenge der Zusammensetzung. Besondere Vorteile liegen in der Verwendung von Siliziumdioxid in Kombination bei einer Creme auf Basis pflanzlichen Öls und/oder Fetts. Vorteilhaft ist die Verwendung von Trihydroxystearin, und/oder Phosphoglyceriden, wie zum Beispiel Lecithinen. Trihydroxystearin wird vorteilhafterweise in einer Menge von 0.001 bis 2.5 Gew.-% bezogen auf die Gesamtmenge der Zusammensetzung eingesetzt und wirkt emulgierend und verdickend. Bevorzugt wird eine Menge mit einer Untergrenze von 0,001 Gew.-% und weiter bevorzugt von 0.01 Gew.-% Trihydroxystearin und einer Obergrenze von 2.0 Gew.-%, weiter bevorzugt von 1.5 Gew.-% und noch weiter bevorzugt von 0.5 Gew.-% Trihydroxystearin verwendet, wobei die Ober- und Untergrenzen frei kombinierbar sind. Polyethylenglycole werden vorteilhafterweise in einer Menge von 0.001-15 Gew.-%, bevorzugt von 3-12 Gew.-% und weiter bevorzugt von 5-9 Gew.-% bezogen auf die Gesamtmenge der Zusammensetzung zugemischt. Besonders bevorzugt werden Polyethylenglycole mit einer Molmasse von 100000-7000000 g/mol, insbesondere von 200000-400000 g/mol. Phosphoglyceride werden vorteilhafterweise in einer Menge von 0.001 bis 3 Gew.-% bezogen auf die Gesamtmenge der Zusammensetzung eingesetzt und wirken emulgierend und erweichend. Bevorzugt wird eine Menge mit einer Untergrenze von 0,001 Gew.-% und weiter bevorzugt von 0.01 Gew.-% Phosphoglycerid und einer Obergrenze von 2 Gew.-%, weiter bevorzugt von 1 Gew.-%, weiter bevorzugt von 0.5 Gew.-% Phosphoglycerid verwendet, wobei die Ober- und Untergrenzen frei kombinierbar sind. Liegen als Stabilisatoren Phosphoglyceride, wie zum Beispiel Lecithin und Trihydroxystearin, gemeinsam vor, kann überraschenderweise die Gesamtmenge an Stabilisatorzusatz verringert werden. Liegen Phosphoglyceride und Trihydroxystearin in Kombination vor, werden vorteilhafterweise Phosphoglyceride in einer Menge von 0.001 bis 3 Gew.-%, weiter bevorzugt in einer Menge von 0.001 bis 2 Gew.-%, besonders bevorzugt in einer Menge von 0.01 bis 1 Gew.-%, und Trihydroxystearin in einer Menge von 0.001 bis 2.5 Gew.-%, besonders bevorzugt in einer Menge von 0.001 bis 1 Gew.-%, und ganz besonders bevorzugt in einer Menge von 0.01 bis 0.5 Gew.-%, bezogen auf die Gesamtmenge der Zusammensetzung verwendet. Phosphoglyceride, insbesondere Lecithin, weiter insbesondere Soyalecithin, scheinen auf die Haftcremezusammensetzung vorwiegend stabilisierend zu wirken. Stearine, insbesondere Trihydroxystearin, erhöhen zudem die Haftungskraft der Creme.

Zusätzlich zu den verwendeten Stabilisatoren aus der Gruppe beinhaltend Siliziumdioxid, Trihydroxystearin und Phosphoglyceride können andere verdickend wirkende Stabilisatoren oder Füllstoffe verwendet werden, wie zum Beispiel Polyethylenglycol oder Talkum. Als verdickend wirkende Stabilisatoren werden Siliziumdioxid oder Trihydroxystearin bevorzugt.

Zweckmässig ist die Kombination der zwei Zusatzstoffe Phosphoglyceriden und Trihydroxystearin, insbesondere Lecithin und Trihydroxystearin, mit zumindest einem weiteren Zusatzstoff oder beiden Zusatzstoffen aus der Gruppe umfassend Siliziumdioxid und Polyethylenglycol.

Besonders bevorzugt ist eine Haftcremezusammensetzung, in welcher zumindest die Zusatzstoffe Phosphoglycerid, Siliziumdioxid, Trihydroxystearin und gegebenenfalls Polyethylenglycol in Kombination vorliegen. Besonders geeignete Phosphoglyceride sind Lecithine.

Ganz besonders bevorzugt ist eine Haftcremezusammensetzung, in welcher zumindest die Zusatzstoffe Lecithin, Siliziumdioxid, Polyethylenglycol und Trihydroxystearin in Kombination vorliegen.

Pflanzliches Öl und/oder Fett ist vorzugsweise zu wenigstens 25 Gew.-%, bevorzugt zu wenigstens 30 Gew.-% bezogen auf die Gesamtmenge der Zusammensetzung in der Haftcreme enthalten und der Fettsäuregehalt des pflanzlichen Öls bzw. Fetts besteht vorzugsweise zu wenigstens 20 Gew.-%, vorzugsweise zu wenigstens 30 Gew.-%, , weiter vorzugsweise mehrheitlich, weiter vorzugsweise zu wenigstens 65 Gew.-% und weiter bevorzugt zu wenigstens 80 Gew.-%, aus höheren Fettsäuren mit einer Kettenlänge von 16-18 C-Atomen.

Im Allgemeinen bestehen die Fettsäuren eines zugegebenen pflanzlichen Öls und/ oder Fetts, d.h. die Gesamtheit der Fettsäuren, zumindest zu 20 Gew.-%, bevorzugt 40 Gew.-%, weiter bevorzugt 50 Gew.-%, vorzugsweise 60 Gew.-%, weiter bevorzugt 70 Gew.-% und meist bevorzugt zumindest zu 80 Gew.-% aus ungesättigten Fettsäuren. Denn dies bringt den Vorteil mit sich, dass die Haftcreme für den Prothesenträger beim Verschlucken besonders verträglich ist.

Vorteilhafterweise setzen sich die genannten höheren Fettsäuren aus einem Anteil von 50 bis 90 Gew.-% Ölsäure und einem Restanteil anderer Fettsäuren mit einer Kettenlänge von 16 bis 18 C-Atomen zusammen. Weiter bevorzugt setzen sich die höheren Fettsäuren aus einem Anteil von 50 bis 90 Gew.-% Ölsäure, einem Anteil von 5 bis 25 Gew.-% Palmitinsäure, und gegebenenfalls einem Restanteil anderer Fettsäuren mit einer Kettenlänge von 16 bis 18 C-Atomen zusammen. Weiter bevorzugt setzen sich die höheren Fettsäuren aus einem Anteil von 50 bis 90 Gew.-% Ölsäure, einem Anteil von 5 bis 25 Gew.-% Palmitinsäure, einem Anteil von 3 bis 25 Gew.-% Linolsäure, und gegebenenfalls einem Restanteil anderer Fettsäuren mit einer Kettenlänge von 16 bis 18 C-Atomen zusammen. Öle wie Olivenöl und Rapsöl fallen unter die genannten Gruppen.

Das pflanzliche Öl und/oder Fett kann zweckmässigerweise in unraffiniertem oder raffiniertem Zustand vorliegen. Vorteilhafterweise liegt das pflanzliche Öl und/oder Fett in einer Menge von 25 bis gegebenenfalls 45 Gew.-% bezogen auf die Gesamtmenge der Zusammensetzung vor. Die Menge an Pflanzenöl bzw. Fett beeinflusst die Konsistenz der Zusammensetzung. Wird zu wenig zugegeben, kann die Creme eine körnig trockene Konsistenz annehmen. Die Zugabe von Siliziumdioxid, Trihydroxystearin, Phophglyceriden und Polyethylenglycolen kann dem entgegenwirken.

Die Cellulosederivate sind wasserlösliche Polymere vorzugsweise ausgewählt aus der Gruppe bestehend aus Methylcellulose, Carboxymethylcellulose, Sodiumcarboxymethylcellulose, Hydroxypropylmethylcellulose und Gemischen davon. Bevorzugt wird Carboxymethylcellulose, insbesondere Natriumcarboxymethylcellulose, verwendet. Das wasserlösliche Polymer ausgewählt aus der Gruppe der Cellulosederivate liegt in einer Menge von 15 bis 45 Gew.-% bezogen auf die Gesamtmenge der Zusammensetzung vor. Vorzugsweise liegt das wasserlösliche Polymer ausgewählt aus der Gruppe der Cellulosederivate in einer Menge von 20 bis 40 Gew.-% und weiter bevorzugt in einer Menge von 25 bis 38 Gew.-% bezogen auf die Gesamtmenge der Zusammensetzung vor.

Vorteilhafterweise liegt das Alkylvinylether/Maleinsäureanhydrid-Copolymer zum Teil als Säure, Ester und/oder Salz vor. Üblicherweise sind die Kationen der Salze ausgewählt aus der Gruppe bestehend aus Calcium-, Kalium-, Natrium-, Magnesium-, Aluminium-, Zinksalzen und Gemischen davon, insbesondere aus der Gruppe bestehend aus Ca²⁺, K+, Na⁺, Mg²⁺, Al³⁺ und/oder Zn²⁺. Als das Alkylvinylether/Maleinsäureanhydrid-Copolymer wird insbesondere ein Methylvinylether/Maleinsäureanhydrid-Copolymer verwendet. Das Methylvinylether/Maleinsäureanhydrid-Copolymer liegt, zum Beispiel als Salz, Ester und/oder Säure, in einer Menge von 20-45 Gew.-% bezogen auf die Gesamtmenge der Zusammensetzung vor. Vorteilhafterweise liegt das Methylvinylether/Maleinsäureanhydrid-Copolymer liegt, zum Beispiel als Salz und/oder Säure, in einer Menge von 25-45 Gew.-% und bevorzugt 25-40 Gew.-% und weiter bevorzugt 28-40 Gew.-% bezogen auf die Gesamtmenge der Zusammensetzung vor.

Zinkverbindungen liegen vorteilhafterweise nicht vor; d.h. ein Zusatz von Zinkverbindungen ist mit Vorteil ausgeschlossen. Insbesondere um gesundheitliche Risiken aufgrund erhöhter Zinkaufnahme durch Haftcremen zu minimieren, sollte der Zinkgehalt auf eine Obergrenze von maximal 1 Gew.-% beschränkt werden. D.h. dass Zink in einer Menge von bis zu 1 Gew.-% bezogen auf die Gesamtmenge der Zusammensetzung enthalten sein kann. Vorteilhafterweise sollte der Zinkgehalt unter einer Obergrenze von 1 Gew.-%, bevorzugt von 0.5 Gew.-%, weiter bevorzugt von 0.1 Gew.-% und weiter bevorzugt von 0.06 Gew.-%, jeweils bezogen auf die Gesamtmenge der Zusammensetzung, liegen. Meist bevorzugt wird jedoch die Abwesenheit jeglichen Zinks oder jeglicher Zinkverbindungen. Gegebenenfalls liegen Zinkverbindungen mit einer Untergrenze von mindestens 0.001 Gew.-%, bevorzugt mindestens 0.01 Gew.-%, weiter bevorzugt mindestens 0.02 Gew.-%, weiter bevorzugt mindestens 0.03 Gew.-%, jeweils bezogen auf die Gesamtmenge der Zusammensetzung vor. Ober- und Untergrenze sind hierbei frei kombinierbar. In Kombination mit den oben erwähnten Ölgehalten kann die Zinkmenge niedrig gehalten werden. Die Untergrenzen ergeben sich aufgrund einer messbaren Wirkung (d.h. Beeinflussung der Hafteigenschaften) oder einer gewünschten Wirkungsstärke infolge des Zinkgehalts.

Haftcremen mit sehr guten Trageigenschaften und Langzeitstabilitätswerten werden vor allem dann erzielt, wenn der oben erwähnte Öl- bzw. Fettgehalt, insbesondere des Olivenöls, eingehalten wird. Die bevorzugten Eigenschaften können alternativ oder zusätzlich durch Verwendung und Optimierung der beschriebenen Stabilisatoren eingestellt werden.

Weitere Zusatzstoffe, wie z.B. Geschmackstoffe, Antioxidantien und Farbstoffe, liegen in Summe in einer Menge von maximal 10 Gew.-%, bevorzugt maximal 2 Gew.-%, und besonders bevorzugt maximal 1 Gew.-%, bezogen auf die Gesamtmenge der Zusammensetzung vor. Mit Hinblick auf die physiologische Unbedenklichkeit wird die Zugabemenge von Zusätzen so gering wie möglich gehalten.

Nachfolgend wird die Erfindung anhand von Beispielen verdeutlicht. Die Beispiele präsentieren Zusammensetzungen, die gute Haftkraft und Haftdauer gewährleisten, insbesondere auch bei Unterdruckbedingungen. Die Prozentangaben sind auf zwei Stellen nach dem Komma gerundet.

### Beispiel 1

| Komponente | Gew.-% |
|---|---|
| Olivenöl ¹⁾ | 40.35 |
| Alkylvinylether/ Maleinsäureanhydrid-Copolymer | 30.26 |
| Carboxymethylcellulose | 27.24 |
| ZnO-Paste ²⁾ | 0.10 |
| Trihydroxystearin | 2.02 |
| Aromazusatzstoff | 0.03 |

| | |
|---|---|
| ¹⁾ kommerziell erhältlich, ²⁾ die Paste besteht aus 50 Gew.-% Öl (z.B. Olivenöl) und 50 Gew.-% Zinkoxid (ZnO), somit sind in Form der ZnO-Paste ca. 0.04 Gew.-% Zink zugegeben. | |

Die Haftcreme gemäss Beispiel 1 hat eine gute Haftungsstärke, die mindestens 12 Stunden anhält. Die Haftcreme hinterlässt einen frischen Geschmack und ein gutes, angenehm seidiges Gefühl im Mund.

### Beispiel 2

| Komponente | Gew.-% |
|---|---|
| Olivenöl ¹⁾ | 31.19 |
| Alkylvinylether/ Maleinsäureanhydrid-Copolymer | 32.22 |
| Carboxymethylcellulose | 36.38 |
| ZnO-Paste ²⁾ | 0.16 |
| Trihydroxystearin | 0.03 |
| Lecithin | 0.02 |
| Aromazusatzstoff ³⁾ | 0.00 |

| | |
|---|---|
| ¹⁾ kommerziell erhältlich, ²⁾ die Paste besteht aus 50 Gew.-% Öl (z.B. Olivenöl) und 50 Gew.-% Zinkoxid (ZnO), somit sind in Form der ZnO-Paste ca. 0.064 Gew.-% Zink zugegeben, ³⁾ die zugegebene Menge liegt unterhalb der Messgenauigkeit, insbesondere unter 0.01 Gew.-%.. | |

Die Haftcreme gemäss Beispiel 2 hat eine gute Haftungsstärke die mindestens 12 Stunden anhält. Die Haftcreme hinterlässt einen frischen Geschmack und ein gutes, angenehm seidiges Gefühl im Mund.

### Beispiel 3

| Komponente | Gew.-% |
|---|---|
| Olivenöl ¹⁾ | 36.99 |
| Alkylvinylether/ Maleinsäureanhydrid-Copolymer | 37.51 |
| Carboxymethylcellulose | 24.91 |
| ZnO-Paste ²⁾ | 0.09 |
| Trihydroxystearin | 0.45 |
| Lecithin | 0.05 |
| Aromazusatzstoff ³⁾ | 0.00 |

| | |
|---|---|
| ¹⁾ kommerziell erhältlich, ²⁾ die Paste besteht aus 50 Gew.-% Öl (z.B. Olivenöl) und 50 Gew.-% Zinkoxid (ZnO), somit sind in Form der ZnO-Paste ca. 0.036 Gew.-% Zink zugegeben, ³⁾ die zugegebene Menge liegt unterhalb der Messgenauigkeit, insbesondere unter 0.01 Gew.-%. | |

Die Haftcreme gemäss Beispiel 3 hat eine gute Haftungsstärke die mindestens 12 Stunden anhält. Die Haftcreme hinterlässt einen frischen Geschmack und ein gutes, angenehm seidiges Gefühl im Mund. Zudem weist diese Haftcreme über Monate hinweg eine gute Langzeitstabilität auf und ist somit lagerfähig.

### Beispiel 4

| Komponente | Gew.-% |
|---|---|
| Olivenöl ¹⁾ | 38.6 |
| Alkylvinylether/Maleinsäureanhydrid-Copolymer | 33 |
| Carboxymethylcellulose | 20 |
| Siliziumdioxid | 0.8 |
| Polyethylenglycol | 7 |
| Trihydroxystearin | 0.4 |
| Lecithin ²⁾ | 0.2 |
| Aromazusatzstoff ³⁾ | - |

| | |
|---|---|
| ¹⁾ kommerziell erhältlich, ²⁾ z.B. Sojalecithin, ³⁾ optional. | |

Die Haftcreme gemäss Beispiel 4 hat eine gute Haftungsstärke die mindestens 12 Stunden anhält. Die Haftcreme hinterlässt einen frischen Geschmack und ein gutes, angenehm seidiges Gefühl im Mund. Zudem bleibt diese Haftcreme auch bei Unterdruckverhältnissen, wie sie in Flugzeugfrachträumen und erhöhten Wohnlagen bestehen stabil.

### Beispiel 5

| Komponente | Gew.-% |
|---|---|
| Olivenöl ¹⁾ | 38.58 |
| Alkylvinylether/Maleinsäureanhydrid-Copolymer | 33.00 |
| Carboxymethylcellulose | 27.00 |
| Trihydxoxystearin | 0.40 |
| Siliziumdioxid ²⁾ | 0.80 |
| Lecithin ³⁾ | 0.20 |
| Aromen | 0.02 |

| | |
|---|---|
| ¹⁾ kommerziell erhältlich, ²⁾ z.B. pyrogenes Siliziumdioxid, ³⁾ z.B. Sojalecithin. | |

Die Haftcreme gemäss Beispiel 5 hat eine gute Haftungsstärke die mindestens 12 Stunden anhält. Die Haftcreme hinterlässt einen frischen Geschmack und ein gutes, angenehm seidiges Gefühl im Mund.

Zusammenfassend wird festgestellt, dass vorteilhafterweise ein pflanzliches Öl und/oder Fett in einer Menge von 25-60 Gew.-%, bevorzugt von 30-45 Gew.-% bezogen auf die Gesamtmenge der Zusammensetzung vorliegt, das wasserlösliche Polymer ausgewählt aus der Gruppe der Cellulosederivate in einer Menge von 10-40 Gew.-%, bevorzugt von 15-38 Gew.-%, weiter bevorzugt von 15-25 Gew.-% bezogen auf die Gesamtmenge der Zusammensetzung vorliegt, das Alkylvinylether/Maleinsäureanhydrid-Copolymer in einer Menge von 25-45 Gew.-% und bevorzugt 28-40 Gew.-% bezogen auf die Gesamtmenge der Zusammensetzung vorliegt, das Siliziumdioxid in einer Menge von 0-2.5 Gew.-% und bevorzugt 0-1.5 Gew.-% bezogen auf die Gesamtmenge der Zusammensetzung vorliegt, das Polyethylenglycol in einer Menge von 0-15 Gew.-% und bevorzugt 0-10 Gew.-% bezogen auf die Gesamtmenge der Zusammensetzung vorliegt, das Trihydroxystearin in einer Menge von bis zu 2.5 Gew.-% und bevorzugt 2.1 Gew.-% bezogen auf die Gesamtmenge der Zusammensetzung vorliegt und das Phosphoglycerid in einer Menge von 0-3 Gew.-% und bevorzugt 0-2 Gew.-% bezogen auf die Gesamtmenge der Zusammensetzung vorliegt. Insbesondere wurde festgestellt, dass das Trihydroxystearin in Kombination mit dem Phosphoglycerid in einer Menge von bis zu 2.5 Gew.-% und bevorzugt bis zu 1 Gew.-% bezogen auf die Gesamtmenge der Zusammensetzung vorliegt. Weiter wurde festgestellt, dass die Gesamtmenge der enthaltenen Trihydroxystearin, Siliziumdioxid und Phosphoglyceride in Summe die Menge von maximal 10 Gew.-%, bevorzugt maximal 5 Gew.-%, weiter bevorzugt maximal 4 Gew.-% bezogen auf die Gesamtmenge der Zusammensetzung bevorzugt nicht übersteigen soll.

## Patentansprüche

1. Zusammensetzung einer Haftcreme enthaltend
a) wenigstens ein Öl oder Fett bzw. Gemische davon, vorzugsweise pflanzlicher Art,
b) wenigstens ein wasserlösliches Polymer ausgewählt aus der Gruppe der Cellulosederivate, und
c) wenigstens ein Alkylvinylether/Maleinsäureanhydrid-Copolymer, **dadurch gekennzeichnet, dass**
d) Trihydroxystearin darin enthalten ist

2. Zusammensetzung nach dem vorangehenden Anspruch **dadurch gekennzeichnet, dass** zumindest eine weitere Substanz ausgewählt aus der Gruppe bestehend aus Siliziumdioxid, Phosphoglyceriden und Polyethylenglycolen darin enthalten ist.

3. Zusammensetzung nach einem der vorangehenden Ansprüche **dadurch gekennzeichnet, dass** Siliziumdioxid in einer Menge von 0.001-5 Gew.-%, bevorzugt von 0.1-4 Gew.-% und weiter bevorzugt von 0.5-3 Gew.-% bezogen auf die Gesamtmenge der Zusammensetzung vorliegt.

4. Zusammensetzung nach einem der vorangehenden Ansprüche **dadurch gekennzeichnet, dass** Trihydroxystearin in einer Menge von 0.001-2.5 Gew.-% und bevorzugt von 0.01-0.5 Gew.-% bezogen auf die Gesamtmenge der Zusammensetzung vorliegt.

5. Zusammensetzung nach einem der vorangehenden Ansprüche dadurch gekerunzeichnet, dass Polyethylenglycole in einer Menge von 0.001-15 Gew.-%, bevorzugt von 3-12 Gew.-% und weiter bevorzugt von 5-9 Gew.-% bezogen auf die Gesamtmenge der Zusammensetzung vorliegen.

6. Zusammensetzung nach einem der vorangehenden Ansprüche **dadurch gekennzeichnet, dass** die Polyethylenglycole eine Molmasse im Bereich von 100000-7000000 g/mol, insbesondere von 200000-400000 g/mol aufweisen.

7. Zusammensetzung nach einem der vorangehenden Ansprüche **dadurch gekennzeichnet, dass** Phosphoglyceride, in einer Menge von 0.001-3 Gew.-%,bevorzugt von 0.01-2 Gew.-% und weiter bevorzugt von 0.1-1 Gew.-% bezogen auf die Gesamtmenge der Zusammensetzung vorliegen.

8. Zusammensetzung nach einem der vorangehenden Ansprüche **dadurch gekennzeichnet, dass** die vorliegenden Phosphoglyceride aus der Gruppe der Lecithine ausgewählt sind, insbesondere Soyalecithin.

9. Zusammensetzung nach einem der vorangehenden Ansprüche **dadurch gekennzeichnet, dass** zumindest ein Lecithin und Trihydroxystearin in Kombination darin vorliegen, gegebenenfalls Lecithin und Trihydroxystearin in Kombination mit zumindest einer weiteren Substanz ausgewählt aus der Gruppe umfassend Siliziumdioxid und Polyethylenglycol vorliegen, und vorteilhafterweise Lecithin in einer Menge von 0.001-3 Gew.-%, vorzugsweise 0.001-2 Gew.-%, besonders bevorzugt 0.01-1 Gew.-%, und Trihydroxystearin in einer Menge von 0.001-2.5 Gew.-%, vorzugsweise 0.001-1 Gew.-%, weiter bevorzugt 0.01-0.5 Gew.-% bezogen auf die Gesamtmenge der Zusammensetzung vorliegen.

10. Zusammensetzung nach einem der vorangehenden Ansprüche **dadurch gekennzeichnet, dass** Siliziumdioxid, Polyethylenglycol und Trihydroxystearin und optional Lecithin in Kombination vorliegen.

11. Zusammensetzung nach einem der vorangehenden Ansprüche **dadurch gekennzeichnet, dass** das pflanzliche Öl bzw. Fett zu wenigstens 25 Gew.-% bezogen auf die Gesamtmenge der Zusammensetzung enthalten ist, wobei der Fettsäuregehalt des pflanzlichen Öls bzw. Fetts mehrheitlich, vorzugsweise zu wenigstens 65 Gew.-%, weiter bevorzugt zu wenigstens 80 Gew.-%, aus höheren Fettsäuren mit einer Kettenlänge von 16-18 C-Atomen besteht.

12. Zusammensetzung nach einem der vorangehenden Ansprüche **dadurch gekennzeichnet, dass** der Fettsäuregehalt des pflanzlichen Öls bzw. Fetts zumindest zu 20 Gew.-%, vorzugsweise 40 Gew.-%, weiter bevorzugt 50 Gew.-%, weiter bevorzugt 60 Gew.-%, weiter bevorzugt 70 Gew.-%, weiter bevorzugt 80 Gew.-% aus ungesättigte Fettsäuren besteht.

13. Zusammensetzung nach einem der vorangehenden Ansprüche **dadurch gekennzeichnet, dass** die Gesamtmenge der enthaltenen Trihydroxystearin, Siliziumdioxid und Phosphoglyceride in Summe die Menge von maximal 10 Gew.-%, bevorzugt maximal 5 Gew.-%, weiter bevorzugt maximal 4 Gew.-% bezogen auf die Gesamtmenge der Zusammensetzung nicht übersteigt.

14. Zusammensetzung nach einem der vorangehenden Ansprüche **dadurch gekennzeichnet, dass** diese bezogen auf die Gesamtmenge der Zusammensetzung
a) 25-60 Gew.% wenigstens eines Öls und/oder Fetts, bevorzugt pflanzlicher Art,
b) 10-40 Gew.-% wenigstens eines wasserlöslichem Polymers ausgewählt aus der Gruppe der Cellulosederivate,
c) 25-45 Gew.-% des wenigstens einen Alkylvinylether/Maleinsäureanhydrid-Copolymers,
d) bis zu 2.5 Gew.-% Trihydroxystearin,
e) 0-15 Gew.-% oder gegebenenfalls bis zu 15 Gew.-% Polyethylenglycole,
f) 0-3 Gew.-% oder gegebenenfalls bis zu 3 Gew.-% Phosphoglyceride,
g) 0-5 Gew.-% oder gegebenenfalls bis zu 5 Gew.-% Siliziumdioxid, und
h) 0-10 Gew.-% weitere Zusatzstoffe
enthält.

15. Zusammensetzung nach einem der vorangehenden Ansprüche **dadurch gekennzeichnet, dass** das pflanzliche Öl bzw. Fett mehrheitlich ein Olivenöl beinhaltet.

## Claims

1. Composition of an adhesive cream containing
a) at least one preferably vegetable oil and/or fat, or mixtures thereof,
b) at least one water-soluble polymer selected from the group of the cellulose derivatives and
c) at least one alkylvinylether/maleic anhydride copolymer,
**characterized in that**
d) trihydroxystearin is contained therein.

2. Composition according to one of the preceding claims, **characterized in that** at least one further substance selected from the group of silicon dioxide, phosphoglycerides and polyethylene glycols is contained therein.

3. Composition according to one of the preceding claims, **characterized in that** silicon dioxide is present in a quantity of 0,001 to 5% by weight, preferably of 0,1 to 4% by weight, more preferably of 0,5 to 3% by weight, based on the total quantity of the composition.

4. Composition according to one of the preceding claims, **characterized in that** trihydroxystearin is present in a quantity of 0,001 to 2,5% by weight, preferably of 0,01 to 0,5% by weight, based on the total quantity of the composition.

5. Composition according to one of the preceding claims, **characterized in that** polyethylene glycols are present in a quantity of 0,001 to 15% by weight, preferably of 3 to 12% by weight and more preferably of 5 to 9% by weight, based on the total quantity of the composition.

6. Composition according to one of the preceding claims, **characterized in that** the polyethylene glycols have a molecular mass in the range of 100000 to 700000 g/mol, in particular of 200000 to 400000 g/mol.

7. Composition according to one of the preceding claims, **characterized in that** phosphoglycerides are present in a quantity of 0,001 to 3% by weight, preferably of 0,01 to 2% by weight and more preferably of 0,1 to 1 % by weight, based on the total quantity of the composition.

8. Composition according to one of the preceding claims, **characterized in that** the present phosphoglycerides are selected from the group of lecithins, in particular soy lecithin.

9. Composition according to one of the preceding claims, **characterized in that** at least one lecithin and trihydroxystearin are present in combination therein, if need be lecithin and trihydroxystearin in combination with at least one further substance selected from the group comprising silicon dioxide and polyethylene glycol and advantageously lecithin in a quantity of 0,001 to 3% by weight, preferably of 0,001 to 2% by weight, more preferably of 0,001 to 1% by weight and trihydroxystearin in a quantity of 0,001 to 2,5% by weight, preferably of 0,001 to 1% by weight, more preferably of 0,01 to 0,5% by weight, based on the total quantity of the composition.

10. Composition according to one of the preceding claims, **characterized in that** silicon dioxide, polyethylene glycol and trihydroxystearin and optionally lecithin are present in combination.

11. Composition according to one of the preceding claims, **characterized in that** the vegetable oil or fat is contained therein for at least 25% by weight, based on the total quantity of the composition, whereby the fatty acid content of the vegetable oil or fat preponderantly consists of at least 65% by weight of higher fatty acids, more preferably of at least 80% by weight of higher fatty acids with a chain length of 16 to 18 carbon atoms.

12. Composition according to one of the preceding claims, **characterized in that** the fatty acid content of the vegetable oil or fat consists of at least 20% by weight, preferably of 40% by weight, more preferably of 50% by weight, more preferably of 60% by weight, more preferably of 70% by weight, more preferably of 80% by weight of unsaturated fats.

13. Composition according to one of the preceding claims, **characterized in that** the total quantity of the contained trihydrostearin, silicon dioxide and phosphoglycerides together does not exceed the quantity of maximally 10% by weight, preferably of 5% by weight, more preferably of 4% by weight, based on the total quantity of the composition.

14. Composition according to one of the preceding claims, **characterized in that** it contains
a) 25 to 60% by weight of at least one preferably vegetable oil and/or fat,
b) 10 to 40% by weight of at least one water soluble polymer selected from the group of the cellulose derivatives,
c) 25 to 45% by weight of the at least one alkylvinylether/maleic anhydride copolymer,
d) up to 2,5% by weight of trihydroxystearin,
e) 0 to 15% by weight or, if need be, up to 15% by weight of polyethylene glycols,
f) 0 to 3% by weight or, if need be, up to 3% by weight of phosphoglycerides,
g) 0 to 5% by weight or, if need be, up to 5% silicon dioxide and
h) 0 to 10% by weight further additives,
based on the total quantity of the composition.

15. Composition according to one of the preceding claims, **characterized in that** the vegetable oil or fat preponderantly contains an olive oil.

## Revendications

1. Composition d'une crème adhésive contenant :
a) au moins une huile et/ou une graisse ou des mélanges de celles-ci, de préférence de type végétal,
b) au moins un polymère soluble dans l'eau sélectionné dans le groupe des dérivés de cellulose,
c) au moins un copolymère d'éther d'alkyivinyle/d'anhydre d'acide maléique, **caractérisée en ce que**
d) de la trihydrosystéarine y est contenue.

2. Composition selon l'une des revendications précédentes, **caractérisée en ce qu'**au moins une autre substance sélectionnée dans le groupe constitué par le dioxyde de silicium, les phosphoglycérides et les polyéthylènes glycols y est contenue.

3. Composition selon l'une des revendications précédentes, **caractérisée en ce que** le dioxyde de silicium est présent en une quantité de 0,001 à 5 % en poids, préférablement de 0,1 à 4 % en poids et plus préférablement de 0,5 à 3 % en poids par rapport à la quantité totale de la composition.

4. Composition selon l'une des revendications précédentes, **caractérisée en ce que** la trihydroxystéarine est présente en une quantité de 0,001 à 2,5 % en poids et préférablement de 0,01 à 0,5 % en poids par rapport à la quantité totale de la composition.

5. Composition selon l'une des revendications précédentes, **caractérisée en ce que** les polyéthylènes glycols sont présents en une quantité de 0,001 à 15 % en poids, préférablement de 3 à 12 % en poids et plus préférablement de 5 à 9 % en poids par rapport à la quantité totale de la composition.

6. Composition selon l'une des revendications précédentes, **caractérisée en ce que** les polyéthylènes glycols présentent une masse moléculaire de l'ordre de 100000 à 700000 g/mol, en particulier de 200000 à 400000 g/mol.

7. Composition selon l'une des revendications précédentes, **caractérisée en ce que** les phosphoglycérides sont présents en une quantité de 0,001 à 3 % en poids, préférablement de 0,01 à 2 % en poids et plus préférablement de 0,1 à 1 % en poids par rapport à la quantité totale de la composition.

8. Composition selon l'une des revendications précédentes, **caractérisée en ce que** les phosphoglycérides présents sont sélectionnés dans le groupe des lécithines, en particulier la lécithine de soja.

9. Composition selon l'une des revendications précédentes, **caractérisée en ce qu'**au moins une lécithine et la trihydroxystéarine y sont présentes en combinaison, le cas échéant la lécithine et la trihydroxystéarine y sont présentes en combinaison avec au moins une autre substance sélectionnée dans le groupe comprenant le dioxyde de silicium et le polyéthylène glycol et avantageusement la lécithine en une quantité de 0,001 à 3 % en poids, de préférence de 0,001 à 2 % en poids, plus préférablement de 0,01 à 1 % en poids et la trihydroxystéarine en une quantité de 0,001 à 2,5 % en poids, préférablement de 0,001 à 1 % en poids, plus préférablement de 0,01 à 0,5 % par rapport à la quantité totale de la composition.

10. Composition selon l'une des revendications précédentes, **caractérisée en ce que** le dioxyde de silicium, le polyéthylène glycol et la trihydrostéarine et en option la lécithine sont présents en combinaison.

11. Composition selon l'une des revendications précédentes, **caractérisée en ce que** l'huile ou la graisse végétale est contenue pour au moins 25 % en poids par rapport à la quantité totale de la composition, la teneur en acide gras de l'huile ou de la graisse végétale étant en majorité, préférablement d'au moins 65 % en poids, plus préférablement d'au moins 80 % en poids d'acides gras supérieurs avec une longueur de chaîne de 16 à 18 atomes de carbone.

12. Composition selon l'une des revendications précédentes, **caractérisée en ce que** la teneur en acide gras de l'huile ou de la graisse végétale est au moins de 20 % en poids, préférablement de 40 % en poids, plus préférablement de 50 % en poids, plus préférablement encore de 70 % en poids, plus préférablement encore de 80 % en poids d'acides gras insaturés.

13. Composition selon l'une des revendications précédentes, **caractérisée en ce que** la quantité totale de la trihydroxystéarine, du dioxyde de silicium et des phosphoglycérides contenus ne dépasse pas en somme la quantité de 10 % en poids maximum, préférablement de 5 % en poids maximum, plus préférablement de 4 % en poids maximum de la quantité totale de la composition.

14. Composition selon l'une des revendications précédentes, **caractérisée en ce que** celle-ci contient, par rapport à la quantité totale de la composition,
a) 25 à 60 % au moins d'une huile et/ou d'une graisse, de préférence végétale,
b) 10 à 40 % en poids d'au moins un polymère soluble dans l'eau sélectionné dans le groupe des dérivés de cellulose,
c) 25 à 45 % en poids du copolymère d'éther d'alkylvinyle/d'anhydre d'acide maléique qui existe au moins,
d) jusqu'à 2,5 % de trihydroxystéarine,
e) 0 à 15 % en poids ou, le cas échéant, jusqu'à 15 % en poids de polyéthylène glycols,
f) 0 à 3 % en poids ou, le cas échéant, jusqu'à 3 % en poids de phosphoglycérides,
g) 0 à 5 % en poids ou, le cas échéant, jusqu'à 5 % en poids de dioxyde de silicium et
h) 0 à 10 % en poids d'autres additifs.

15. Composition selon l'une des revendications précédentes, **caractérisée en ce que** l'huile ou la graisse végétale contient en majorité de l'huile d'olive.
